# EUROPEAN PATENT APPLICATION

(11) **EP 4 775 138 A1**
(43) Date of publication of application: **15.07.2026**
(21) Application number: 25781585.2
(22) Date of filing: 26.03.2025
(51) Int. Cl.: A61B 5/02

(54) **DETECTION MODULE AND WEARABLE DEVICE**

(30) Priority: 01.04.2024 CN 202410390622
(71) Applicant: HUAWEI TECHNOLOGIES CO., LTD., Shenzhen 518129 (CN)
(72) Inventor: REN, Jingxuan, Shenzhen, Guangdong 518129 (CN); JIN, Junye, Shenzhen, Guangdong 518129 (CN); ZHAO, Menglong, Shenzhen, Guangdong 518129 (CN)
(74) Representative: Isarpatent
(86) International application number: PCT/CN2025/085183
(87) International publication number: WO 2025/209288

(57) **Abstract**

A detection assembly (4) and a wearable device (1) are disclosed. The detection assembly (4) includes a cover (45), a coherent light source (46), and an image sensor (47). The cover (45) is disposed on a side wall (31) of a housing (3) of the wearable device (1). The cover (45) has an outer surface (45x) and an inner surface (45y) opposite to the outer surface (45x), and the outer surface (45x) is exposed from the side wall (31). The cover (45) has a first light-transmitting region (45a) and a second light-transmitting region (45c). A coherent light source (46) is disposed on a side of the inner surface (45y) of the cover (45), and the coherent light source (46) is configured to emit coherent light that passes through the first light-transmitting region (45a). The image sensor (47) is disposed on the side of the inner surface (45y) of the cover (45), and the image sensor (47) is configured to perform imaging on light that passes through the second light-transmitting region (45c).

## Description

This application claims priority to Chinese Patent Application No. 202410390622.3, filed with the China National Intellectual Property Administration on April 1, 2024 and entitled "DETECTION ASSEMBLY AND WEARABLE DEVICE", which is incorporated herein by reference in its entirety.

### TECHNICAL FIELD

This application relates to the field of wearable devices, and in particular, to a detection assembly and a wearable device.

### BACKGROUND

Currently, some detection functions, such as physiological feature monitoring, posture detection, and motion detection, have been integrated into wearable devices such as a smartwatch and a smart band. As user requirements continuously increase, users expect wearable products to have more abundant functions while ensuring that they remain lightweight and portable. Therefore, how to implement miniaturization while ensuring performance of the wearable device becomes an urgent problem to be resolved.

### SUMMARY

Embodiments of this application provide a detection assembly and a wearable device, so that more functions can be integrated into a small-sized product like the wearable device.

According to a first aspect, embodiments of this application provide a detection assembly. The detection assembly includes a cover, a coherent light source, and an image sensor. The cover is configured to be disposed on a side wall of a housing of the wearable device. The cover has an outer surface and an inner surface opposite to the outer surface, and the outer surface is exposed from the side wall. The cover has a first light-transmitting region and a second light-transmitting region. A coherent light source is disposed on a side of the inner surface of the cover, and the coherent light source is configured to emit coherent light that passes through the first light-transmitting region. The image sensor is disposed on the side of the inner surface of the cover, and the image sensor is configured to perform imaging on light that passes through the second light-transmitting region.

In this embodiment, the cover is disposed on the side wall of the wearable device, so that the detection assembly may be integrated on the side wall. When human body tissue touches the cover, coherent light may be emitted into the human body tissue through the first light-transmitting region of the cover, and is scattered by the human body tissue to the image sensor to form an image. The image sensor may detect a laser Doppler effect caused by blood flow, so that a blood flow change in a pulse fluctuation process can be detected, and blood flow information can be monitored. In addition, the image generated by the image sensor is processed, so that other physiological feature information may be further generated. Therefore, the coherent light source and the image sensor are disposed in the wearable device, so that a large quantity of physiological feature monitoring functions such as blood flow information monitoring can be integrated into a small-sized product like the wearable device. In addition, the detection assembly is disposed on the side wall of the wearable device, so that the detection assembly and a button of the wearable device can be disposed together, to facilitate a user operation and enhance user experience. In addition, the user can use a finger to touch the cover to implement physiological feature monitoring, improving physiological feature monitoring accuracy.

In an implementation of the first aspect, there are at least two coherent light sources, and the at least two coherent light sources are configured to separately transmit coherent light of different wavelengths. A plurality of coherent light sources are disposed, and coherent light of different bands may be used to monitor blood flow information and other different physiological feature information, enhancing detection performance of the detection assembly.

In an implementation of the first aspect, the detection assembly further includes a light-emitting diode, where the light-emitting diode is disposed on the side of the inner surface of the cover, and the light-emitting diode is configured to emit incoherent light that passes through the first light-transmitting region; and the image sensor is further configured to perform imaging on light that passes through the second light-transmitting region. In this embodiment, the incoherent light emitted by the light-emitting diode may enter the human body tissue after passing through the first light-transmitting region. After being scattered by the tissue, the incoherent light may pass through the second light-transmitting region and be collected by the image sensor. After the image generated by collecting the incoherent light by the image sensor is processed by the controller, another physiological feature like a heart rate, blood oxygen, blood pressure, breathing, and/or emotion may be obtained. After the light-emitting diode is disposed, a small quantity of coherent light sources may be disposed. In comparison with a solution in which blood flow information and another physiological feature are monitored by using a plurality of coherent light sources, in this embodiment, a high-cost and high-power coherent light source is replaced by a low-cost and low-power light-emitting diode, thereby reducing costs and power consumption. In addition, a light source combining the coherent light source and the light-emitting diode may also increase flexibility of a structure layout, to meet a product requirement.

In an implementation of the first aspect, the detection assembly further includes a light-blocking structure, where the light-blocking structure is disposed between a light source and the image sensor, the light-blocking structure is configured to block light that is emitted by the light source and that does not pass through the outer surface of the cover from entering the image sensor, and the light source includes the coherent light source, or includes the coherent light source and the light-emitting diode. In this embodiment, the light-blocking structure may prevent the light emitted by the coherent light source from reaching the image sensor from an inner side of the cover or through the inside of the cover. It can be ensured that the coherent light can be collected by the image sensor after being scattered by finger tissue but the coherent light does not directly enter the image sensor, ensuring detection precision of the physiological feature information.

In an implementation of the first aspect, the light-blocking structure includes an opaque region of the cover. The opaque region is disposed on the cover, so that the light emitted by the coherent light source can be prevented from directly reaching the image sensor through the cover, to ensure detection precision of the physiological feature information.

In an implementation of the first aspect, the detection assembly further includes a pressure sensor, and the pressure sensor is disposed on the side of the inner surface of the cover. The pressure sensor is disposed, so that contact pressure exerted by the user on the cover can be detected, and comprehensive processing is performed on the contact pressure and the image generated by the image sensor, to eliminate interference caused by unstable pressing to imaging of the image sensor, reduce or avoid impact of motion artifacts caused by unstable pressing on physiological feature monitoring, and improve detection precision of the physiological feature information.

In an implementation of the first aspect, the detection assembly further includes a force conductive structure, the force conductive structure is disposed between the cover and the pressure sensor, and the force conductive structure is configured to conduct, to the pressure sensor, contact pressure exerted on the cover. In this embodiment, when the arrangement of the cover and the pressure sensor is designed based on a structural layout requirement, the contact pressure exerted on the cover may be conducted to the pressure sensor through the force conductive structure, to detect the contact pressure.

In an implementation of the first aspect, the detection assembly further includes a circuit board disposed on the side of the inner surface of the cover, where the pressure sensor, the light source, and the image sensor are all electrically connected to the circuit board, and the light source includes the coherent light source, or includes the coherent light source and the light-emitting diode. The pressure sensor, the light source, and the image sensor are disposed on the same circuit board, so that a structural layout can be simplified, and a volume and a weight of the detection assembly can be reduced, to meet a product requirement.

In an implementation of the first aspect, the light source and the image sensor are disposed on one side of a thickness direction of the circuit board, and the pressure sensor is disposed on the other side of the thickness direction of the circuit board. The light source/image sensor and the pressure sensor are separately disposed on two opposite sides of the circuit board, so that a device layout on a first circuit board can be simplified.

In an implementation of the first aspect, the force conductive structure includes a force conductive member. The force conductive member includes a force conductive plate and a force conductive rod. The force conductive rod is convexly disposed on a plate surface of the force conductive plate. The image sensor, the force conductive plate, the force conductive rod, and the pressure sensor are sequentially arranged. The force conductive plate is configured to carry the image sensor and the coherent light source. The force conductive rod is connected to the pressure sensor. The force conductive member is disposed, so that a structural layout requirement of the cover and the pressure sensor can be matched. The contact pressure exerted on the cover is conducted to the pressure sensor through the force conductive member, so that the contact pressure is detected, the image sensor can be carried, and structural reliability of the detection assembly is ensured.

In an implementation of the first aspect, the force conductive structure further includes a fastener, where the fastener includes a first wall, a second wall, and a third wall. The first wall, the second wall, and the third wall are sequentially connected. Both the first wall and the third wall form a bending angle with the second wall. The first wall and the third wall are located on a same side of a thickness direction of the second wall. The force conductive rod, the pressure sensor, and the second wall are sequentially disposed. Both the force conductive rod and the pressure sensor are located between the first wall and the third wall. The pressure sensor is connected to the second wall. Both the first wall and the third wall are configured to be connected to the housing. The fastener is designed, so that the pressure sensor, the force conductive member, the image sensor, and the like may be fastened to the housing, to ensure assembly reliability of the detection assembly and the side wall.

In an implementation of the first aspect, the detection assembly further includes a first circuit board and a second circuit board. Both the first circuit board and the second circuit board are disposed on the inner side of the cover. Both the coherent light source and the image sensor are disposed on the first circuit board, and the pressure sensor is disposed on the second circuit board. Two circuit boards are disposed, and the light source/image sensor and the pressure sensor are separately disposed on different circuit boards, so that a requirement of a structural layout of the wearable device and a requirement of a stacked layout of electrical devices can be met.

In an implementation of the first aspect, the detection assembly further includes an ambient light sensor, where the ambient light sensor is disposed on the side of the inner surface of the cover; and the ambient light sensor is configured to detect ambient light that passes through the second light-transmitting region; or the cover has a third light-transmitting region, and the ambient light sensor is configured to detect ambient light that passes through the third light-transmitting region. The ambient light sensor is disposed, so that a parameter of ambient light in an external environment can be detected, and the wearable device can provide information like sunshine duration, a sun protection suggestion, a sunshine suggestion, and a vision protection suggestion for the user. For example, the parameter of the ambient light may include but is not limited to an ultraviolet radiation amount. Because the ambient light sensor is disposed on the side wall of the wearable device, when the user normally wears the wearable device, the ambient light sensor can detect the ambient light, and the wearable device does not need to be removed from a human body. Therefore, an operation can be simplified, and user experience can be improved. In addition, the ambient light sensor, the light source, and the image sensor are all disposed on the side wall, so that the wearable device can detect both the ambient light and the physiological feature.

In an implementation of the first aspect, the detection assembly further includes a light filter, the light filter is disposed in the second light-transmitting region or the third light-transmitting region, and the light filter corresponds to the ambient light sensor. The light filter may allow light of some bands to pass through and filter light of other bands. In this embodiment, an ambient light sensor that matches the light filter may be selected, to meet a product requirement.

In an implementation of the first aspect, the light source, the image sensor, and the ambient light sensor are sequentially disposed, where the light source includes the coherent light source, or includes the coherent light source and the light-emitting diode. A specific distance between the ambient light sensor and the light source is kept, so that a risk that light of the light source directly enters the ambient light sensor can be reduced, and detection precision of the ambient light sensor can be ensured.

In an implementation of the first aspect, the detection assembly further includes a lens component, and the lens component is disposed in the second light-transmitting region. The lens component is configured to project the scattered light to the image sensor, to implement an optical path and an imaging effect required for design.

In an implementation of the first aspect, the lens component is disposed on the side of the inner surface of the cover. The lens component is disposed on the side of the inner surface of the cover, so that the lens component can be protected, and it can be further ensured that an appearance of the wearable device is concise.

In an implementation of the first aspect, the detection assembly further includes an electrically conductive layer and an electrically conductive member; the electrically conductive layer is disposed on the outer surface of the cover, the inner surface of the cover, and a side surface connecting the outer surface and the inner surface; and the electrically conductive member is disposed on the side of the inner surface of the cover, and the electrically conductive member is connected to a region that is of the electrically conductive layer and that is located on the inner surface. The electrically conductive layer may be used as a detection electrode, and is configured to detect a bioelectric signal when being touched by a human body. The detection electrode covering the surface of the cover is designed, so that an electrode structure can be simplified, and lightness and thinness of the wearable device are implemented. The electrically conductive member may conduct bioelectricity to a corresponding circuit in the wearable device, to process the bioelectric signal.

According to a second aspect, an embodiment of this application provides a wearable device, including a housing and the detection assembly according to any one of the foregoing implementations. The housing includes a side wall, the detection assembly is disposed on the side wall, and a cover of the detection assembly is exposed from the side wall.

In this embodiment, the detection assembly may be integrated on the side wall. When human body tissue touches the cover, coherent light may be emitted into the human body tissue through the first light-transmitting region of the cover, and is scattered by the human body tissue to the image sensor to form an image. The image sensor may detect a laser Doppler effect caused by blood flow, so that a blood flow change in a pulse fluctuation process can be detected, and blood flow information can be monitored. In addition, the image generated by the image sensor is processed, so that other physiological feature information may be further generated. Therefore, the coherent light source and the image sensor are disposed in the wearable device, so that a large quantity of physiological feature monitoring functions such as blood flow information monitoring can be integrated into a small-sized product like the wearable device. In addition, the detection assembly is disposed on the side wall of the wearable device, so that the detection assembly and a button of the wearable device can be disposed together, to facilitate a user operation and enhance user experience. In addition, the user can use a finger to touch the cover to implement physiological feature monitoring, improving physiological feature monitoring accuracy.

In an implementation of the second aspect, the wearable device further includes a display, the display is disposed on the housing, and the side wall surrounds the display. The solution in this embodiment may be applied to a wearable device having a display, so that more functions are integrated into the device.

In an implementation of the second aspect, the wearable device further includes a controller, the controller is disposed in the housing, and the controller is configured to: control a coherent light source in the detection assembly to emit light, control the image sensor to perform imaging on light that passes through the second light-transmitting region, process an image generated by the image sensor, and generate blood flow information. The wearable device in this embodiment may implement detection of blood flow information, enhancing performance of the wearable device.

In an implementation of the second aspect, the controller is further configured to: control a light-emitting diode in the detection assembly to emit light, control the image sensor to perform imaging on the light that passes through the second light-transmitting region, process the image generated by the image sensor, and generate other physiological feature information. The wearable device in this embodiment may implement detection of other physiological feature information, enhancing performance of the wearable device.

In an implementation of the second aspect, the controller is specifically configured to: perform sliding window processing on the image generated by the image sensor, and generate blood flow information and other physiological feature information. The sliding window processing is used to improve processing precision of image data, ensuring detection precision of the physiological feature information.

In an implementation of the second aspect, the controller is further configured to: process the image generated by the image sensor, and generate identity recognition information. In this embodiment, identity recognition may be implemented by using the light source and the image sensor, enhancing performance of the wearable device, and eliminating the need of an additional identity recognition module.

According to a third aspect, an embodiment of this application provides a detection method, including: receiving a detection instruction; controlling, based on the detection instruction, a light source in a detection assembly to emit light, where the light source includes a coherent light source or includes a coherent light source and a light-emitting diode; controlling an image sensor to perform imaging on light that passes through a second light-transmitting region; and processing an image generated by the image sensor, and generating blood flow information and other physiological feature information. The solution in this embodiment can implement detection of the physiological feature information, and improve performance of a device.

According to a fourth aspect, an embodiment of this application provides an electronic device, including: one or more controllers, and a non-transitory computer-readable storage medium that is coupled to the controller and stores a program executed by the controller. When the program is executed by the controller, the electronic device performs the detection method. The solution in this embodiment can implement detection of physiological feature information, and improve performance of an electronic device.

According to a fifth aspect, an embodiment of this application provides a non-transitory computer-readable storage medium, including program code. When the program code is executed by a computer device, the computer device is enabled to perform the detection method. The solution in this embodiment may enable the computer device to monitor physiological feature information.

According to a sixth aspect, an embodiment of this application provides a chip, including: a controller, configured to invoke a computer program from a memory and run the computer program, so that a device in which the chip is mounted performs the detection method. The solution in this embodiment may enable the device to monitor physiological feature information.

### BRIEF DESCRIPTION OF DRAWINGS

FIG. 1 is a diagram of a three-dimensional structure of a wearable device according to an embodiment of this application;
FIG. 2 is a diagram of a side-view structure of the wearable device in FIG. 1;
FIG. 3 is a diagram of an exploded structure of the wearable device in FIG. 1;
FIG. 4 is a diagram of a structure of a housing of the wearable device in FIG. 3 from an angle of view;
FIG. 5 is a diagram of a structure of a housing of the wearable device in FIG. 3 from another angle of view;
FIG. 6 is a diagram of a partially enlarged structure of a location B in FIG. 5;
FIG. 7 is a diagram of an assembly structure of a detection assembly of the wearable device in FIG. 3;
FIG. 8 is a diagram of an exploded structure of the detection assembly in FIG. 7;
FIG. 9 is a diagram of an assembly structure of an electronic component of the detection assembly in FIG. 8;
FIG. 10 is a diagram of an overall exploded structure of the electronic component in FIG. 9;
FIG. 11 is a diagram of an exploded structure of a part of the electronic component in FIG. 9;
FIG. 12 is a diagram of a structure of a force conductive member or the like in FIG. 11;
FIG. 13 is a diagram of a sectional view structure based on a C-C section of the detection assembly in FIG. 7;
FIG. 14 is a diagram of an assembly structure of a detection assembly and a housing;
FIG. 15 is a diagram of a partially enlarged structure of a location D in FIG. 14;
FIG. 16 is a diagram of a detection principle of a detection assembly;
FIG. 17 is a diagram of a cross-sectional structure of a detection assembly according to an embodiment;
FIG. 18 is a diagram of a cross-sectional structure of a detection assembly according to an embodiment; and
FIG. 19 is a diagram of a layout structure of a coherent light source, a light-emitting diode, an ambient light sensor, and an image sensor of a detection assembly according to an embodiment.

### DESCRIPTION OF EMBODIMENTS

An embodiment of this application provides a wearable device. The wearable device includes but is not limited to a smartwatch, a smart band, smart glasses (such as virtual reality glasses and augmented reality glasses), a smart helmet, an electronic blood pressure meter, a headset, a sound box, mobile Wi-Fi, smart clothing, a smart backpack, a bracelet, a ring, a cane, and the like. The wearable device may have a display, or may not have a display.

The wearable device has a housing, and the housing may be a single component or a part formed by assembling a plurality of components. The housing may include an outer housing that forms an outer surface of the wearable device, and may further include an inner housing inside the outer housing. The housing has a side wall, and the side wall includes a part that is exposed to the outside and that forms the outer surface of the wearable device.

The side wall may be a housing wall surrounding a front surface of the wearable device (a surface facing a user when the wearable device is normally used), and a thickness direction of the side wall may be approximately parallel to the front surface. For the wearable device having the display, like the smartwatch or the smart band, a front surface is a surface on which the display is located, and a side wall may be a housing wall surrounding the display. For the wearable device without the display, a front surface may be a main user interface of the wearable device, and a side wall may be a housing wall surrounding the main user interface.

Alternatively, a thickness direction axis of the wearable device may be defined, and the thickness direction axis is used to define a thickness direction of the wearable device. For the wearable device having the display, this thickness direction axis may be approximately perpendicular to the display. For the wearable device without the display, this thickness direction axis may be approximately perpendicular to the main user interface of the wearable device. The side wall is a housing wall surrounding the thickness direction axis.

The following uses an example in which the wearable device is a smartwatch for description.

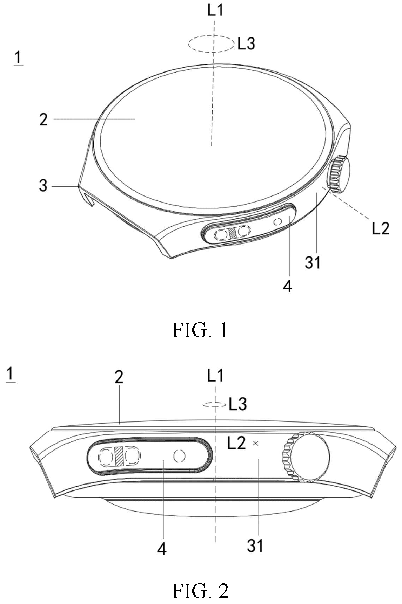

FIG. 1, FIG. 2, and FIG. 3 are diagrams of structures of a wearable device 1 according to an embodiment. As shown in FIG. 1 to FIG. 3, the wearable device 1 may include a housing 3, a display 2, a detection assembly 4, and the like.

As shown in FIG. 1 and FIG. 2, the housing 3 has a side wall 31, and the side wall 31 may surround a thickness direction axis L1 of the wearable device 1. The display 2 is mounted on the housing 3, the side wall 31 also surrounds the display 2, and a thickness direction L2 of the side wall 31 may be approximately parallel to the display 2. In a view of FIG. 2, the thickness direction L2 may be approximately perpendicular to the feature, and a symbol × is used to represent the thickness direction L2. The detection assembly 4 is mounted on the side wall 31.

It may be understood that the wearable device 1 may further include a circuit board and various devices electrically connected to the circuit board. The circuit board may include a main board, and may further include a sub-board electrically connected to the main board. The circuit board may include a rigid circuit board, and may further include a flexible circuit board. The device includes but is not limited to one or more of a controller (for example, an application processor or a micro control unit), an audio device, a camera module, a battery, and the like.

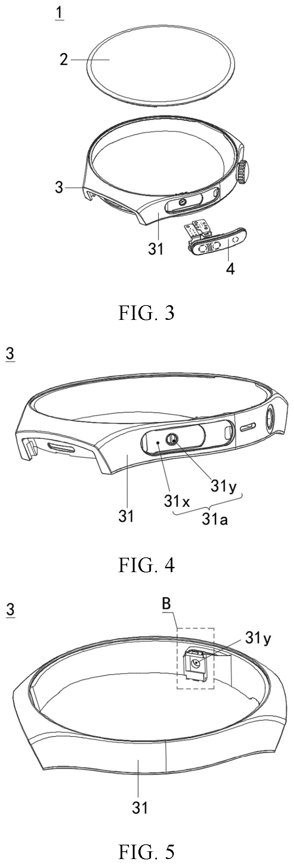

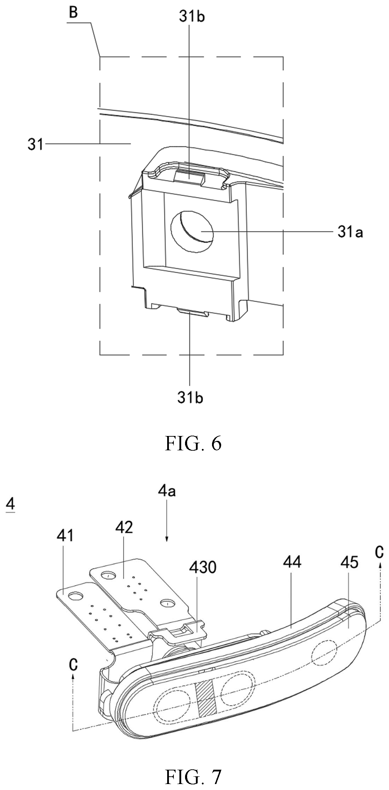

FIG. 4 and FIG. 5 show examples of structures of the housing 3. As shown in FIG. 3 and FIG. 4, a through hole 31a may be disposed on the side wall 31. The through hole 31a may be, for example, a stepped hole. A large hole 31x in the stepped hole may be close to an outer side of the housing 3, and a small hole 31y may be close to an inner side of the housing 3. In another embodiment, the through hole 31a may alternatively be a straight hole whose aperture is basically unchanged. The through hole 31a is configured to mount the detection assembly 4, and the detection assembly 4 may also be referred to as a side button assembly. It may be understood that a person skilled in the art may dispose a through hole 31a based on a structure of a side wall. This is not limited to the examples shown in FIG. 4 and FIG. 5. For example, the through hole 31a may be a single through hole. FIG. 6 is a diagram of a partially enlarged structure of a location B in FIG. 5. As shown in FIG. 6, a buckle 31b may be disposed on an inner side of the side wall 31. There are, for example, two buckles 31b. The buckle 31b is configured to fasten the detection assembly 4 (which is described below).

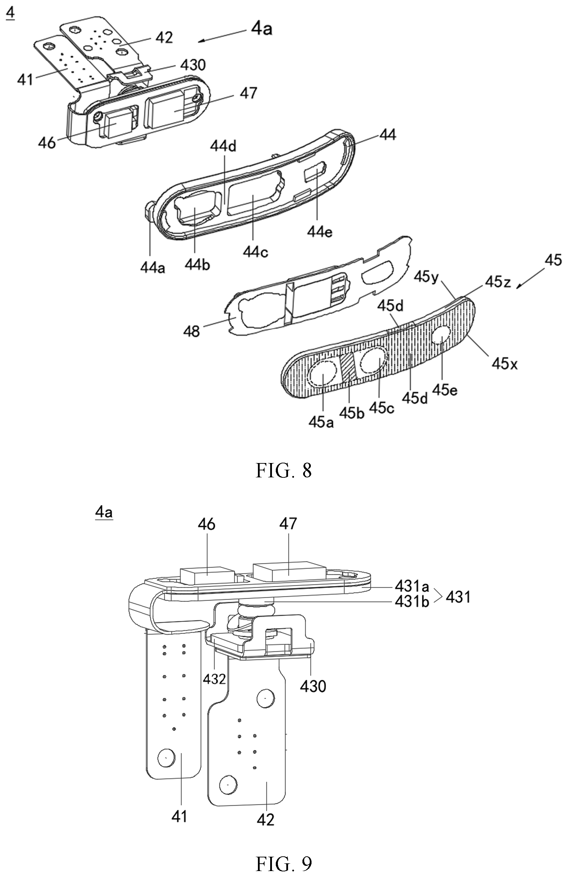

FIG. 7 and FIG. 8 show examples of structures of the detection assembly 4. As shown in FIG. 7 and FIG. 8, the detection assembly 4 may include a support 44, a cover 45, an electronic component 4a, and the like.

As shown in FIG. 8, for example, the support 44 may be approximately of a plate structure, and an outline of the support 44 may be approximately an elliptical runway. A through hole 44b and a through hole 44c may be disposed on the support 44. The support 44 may further have a connection structure 44a, and the connection structure 44a may be, for example, a buckle.

As shown in FIG. 8, for example, the cover 45 may be approximately of a plate structure, and an outline of the cover 45 may be approximately an elliptical runway.

As shown in FIG. 8, the cover 45 has an outer surface 45x and an inner surface 45y. The outer surface 45x and the inner surface 45y are disposed opposite to each other. In other words, the outer surface 45x and the inner surface 45y face away from each other. When the detection assembly 4 is mounted on the side wall 31, the outer surface 45x is exposed from the side wall 31, the outer surface 45x faces the outside of the wearable device 1, and the inner surface 45y faces the inside of the wearable device 1. In this embodiment of this application, one side of the outer surface 45x of the cover 45 may be referred to as an outer side of the cover 45, and one side of the inner surface 45y of the cover 45 may be referred to as an inner side of the cover 45.

As shown in FIG. 8, the cover 45 may have a first light-transmitting region 45a and a second light-transmitting region 45c, and both the first light-transmitting region 45a and the second light-transmitting region 45c can transmit light.

Both the first light-transmitting region 45a and the second light-transmitting region 45c may be three-dimensional regions. There may be a specific distance between the two light-transmitting regions. Shapes of the two light-transmitting regions are not limited. For example, the two light-transmitting regions may be cylindrical regions. In FIG. 8, a dashed line indicates an end face of the cylindrical region.

Both the first light-transmitting region 45a and the second light-transmitting region 45c may be entity structures. The two light-transmitting regions may be integrally formed in the cover 45. The two light-transmitting regions are connected to another region of the cover 45, and may be formed in a manufacturing process of the cover 45. Alternatively, the two light-transmitting regions may be manufactured in advance (the two light-transmitting regions are parts), and are assembled to a body of the cover through an assembly process, to manufacture the cover 45.

Alternatively, at least one of the first light-transmitting region 45a and the second light-transmitting region 45c may be a through hole.

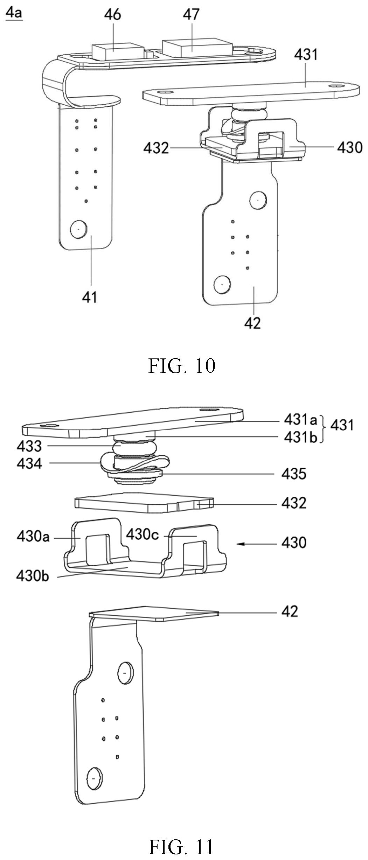

FIG. 9 and FIG. 10 show examples of structures of the electronic component 4a. As shown in FIG. 9 and FIG. 10, the electronic component 4a may include a coherent light source 46 and an image sensor 47. The coherent light source 46 and the image sensor 47 may be arranged adjacently and side by side. With reference to FIG. 9 and FIG. 1, the coherent light source 46 and the image sensor 47 may be disposed, for example, in a circumferential direction L3 of the wearable device 1.

The coherent light source 46 is configured to emit coherent light having substantially the same frequency, substantially the same direction of vibration, and substantially constant phase difference. The coherent light source 46 includes but is not limited to a laser, for example, a vertical-cavity surface-emitting laser (vertical-cavity surface-emitting laser, VCSEL). There may be one or more coherent light sources 46. When there are a plurality of coherent light sources 46, wavelengths of light emitted by the coherent light sources 46 may be different from each other. In other words, wavelengths of the coherent light sources 46 may be different from each other. For example, wavelengths of the plurality of coherent light sources 46 may include a near-infrared light band, a red light band, a blue light band, a yellow light band, and a green light band.

The image sensor 47 is configured to convert an optical signal into an image signal, to implement imaging. The image sensor 47 may include a plurality of photosensitive elements, and the photosensitive elements form a two-dimensional imaging array. The photosensitive element includes but is not limited to a complementary metal oxide semiconductor (complementary metal-oxide-semiconductor, CMOS), a photodiode, and the like.

As shown in FIG. 10, in an embodiment, the electronic component 4a may further include a first circuit board 41, and the first circuit board 41 may be, for example, a flexible circuit board or a rigid circuit board. The first circuit board 41 may be electrically connected to the sub-board or the main board of the wearable device 1. Both the coherent light source 46 and the image sensor 47 are electrically connected to the first circuit board 41. For example, both the coherent light source 46 and the image sensor 47 may be welded to the first circuit board 41. Both the coherent light source 46 and the image sensor 47 may be electrically connected to the controller on the main board through the first circuit board 41.

As shown in FIG. 10 and FIG. 11, in an embodiment, the electronic component 4a may further include a pressure sensor 432. The pressure sensor 432 is configured to sense contact pressure on the cover 45 when a human body touches the cover 45, and convert the contact pressure into an electrical signal, so that contact pressure detection (which is further described below) is implemented. For example, the pressure sensor 432 may work based on a capacitance principle. When there is contact pressure, a distance between two metal plates in the pressure sensor 432 changes, causing a capacitance change between the metal plates. This change may be detected by an internal circuit of the pressure sensor 432, and converted into a voltage signal or a current signal for output.

As shown in FIG. 10 and FIG. 11, in an embodiment, the electronic component 4a may further include a second circuit board 42. The second circuit board 42 may be, for example, a flexible circuit board or a rigid circuit board. The second circuit board 42 may be electrically connected to the sub-board or the main board of the wearable device 1. The pressure sensor 432 may be electrically connected to the second circuit board 42, and may be electrically connected to the controller on the main board through the second circuit board 42.

With reference to FIG. 8 to FIG. 10, in an embodiment, to meet a structural layout, the pressure sensor 432 may be far away from the cover 45. To conduct contact pressure between the cover 45 and the pressure sensor 432, the detection assembly 4 may further include a force conductive structure.

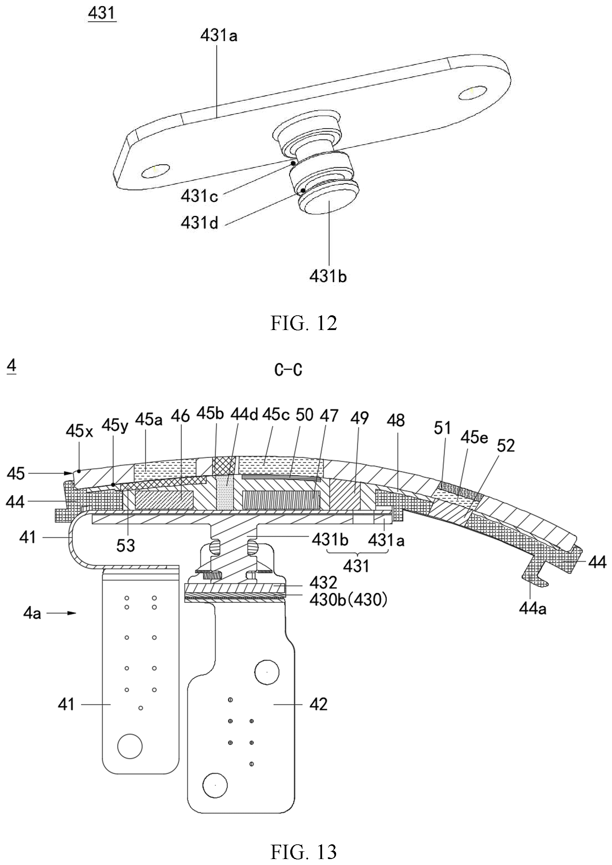

For example, as shown in FIG. 11 and FIG. 12, the force conductive structure may include a force conductive member 431, and the force conductive member 431 may include a force conductive plate 431a and a force conductive rod 431b that are connected to each other. The force conductive plate 431a may be of a plate structure, and the force conductive rod 431b may be approximately of a bar structure. An axis of the force conductive rod 431b may be approximately in a thickness direction of the force conductive plate 431a. In other words, the force conductive rod 431b is convexly disposed on a plate surface of the force conductive plate 431a. A normal line of the plate surface is approximately in the thickness direction of the force conductive plate 431a. In other words, the plate surface is approximately perpendicular to the thickness direction of the force conductive plate 431a.

As shown in FIG. 12, in an embodiment, a groove 431c and a groove 431d may be disposed on the force conductive rod 431b, and both the groove 431c and the groove 431d may surround the axis of the force conductive rod 431b. With reference to FIG. 12 and FIG. 11, in an embodiment, the force conductive structure may further include a sealing ring 433, a limiting ring 434, and a washer 435. The sealing ring 433 may be mounted in the groove 431c, and the limiting ring 434 and the washer 435 may be mounted in the groove 431d. The sealing ring 433 is configured to implement sealing between the force conductive rod 431b and the side wall 31. The limiting ring 434 may abut against the side wall 31, and the limiting ring 434 and the washer 435 may jointly limit the force conductive rod 431b on the side wall 31, to prevent the force conductive rod 431b from being detached from the side wall 31. The foregoing assembly and cooperation are further described below. In another embodiment, no groove may be disposed on the force conductive rod 431b, and the sealing ring 433, the limiting ring 434, the washer 435, and the like may not be mounted on the force conductive rod 431b.

With reference to FIG. 10 and FIG. 11, the image sensor 47, a part that is on the first circuit board 41 and on which the image sensor 47 is disposed, the force conductive plate 431a, the force conductive rod 431b, and the pressure sensor 432 may be sequentially disposed. The force conductive plate 431a may be connected to the part that is on the first circuit board 41 and on which the image sensor 47 is disposed, and the force conductive plate 431a may support and carry the image sensor 47. It may be understood that the force conductive plate 431a also supports and carries the coherent light source 46. The force conductive rod 431b is in contact with the pressure sensor 432. The contact pressure may be conducted from the cover 45 to the pressure sensor 432 through the force conductive member 431. This is further described below.

As shown in FIG. 10 and FIG. 11, in an embodiment, the force conductive structure may further include a fastener 430. For example, the fastener 430 may include a first wall 430a, a second wall 430b, and a third wall 430c that are sequentially connected. The first wall 430a and the third wall 430c are disposed opposite to each other, and the second wall 430b is connected to the first wall 430a and the third wall 430c. The first wall 430a is bent relative to the second wall 430b, and the first wall 430a and the second wall 430b form a bending angle, where the bending angle is, for example, approximately 90 degrees. The third wall 430c is bent relative to the second wall 430b, and the third wall 430c and the second wall 430b form a bending angle, where the bending angle is, for example, approximately 90 degrees. Both the first wall 430a and the third wall 430c may bend toward a same side of a thickness direction of the second wall 430b.

As shown in FIG. 10 and FIG. 11, the force conductive rod 431b, the pressure sensor 432, and the second wall 430b may be sequentially disposed. Both the force conductive rod 431b and the pressure sensor 432 may be located between the first wall 430a and the third wall 430c. The pressure sensor 432 may be connected to the second wall 430b. The second wall 430b may support and carry the pressure sensor 432 and the force conductive rod 431b. In the following descriptions, the first wall 430a and the third wall 430c may be fastened to the housing 3, so that the fastener 430 can fasten the pressure sensor 432 and the force conductive member 431 to the housing 3, to avoid shaking of a force conductive structure with a large volume and weight, and ensure assembly reliability and structural strength of the force conductive structure. In another embodiment, based on a product structure design solution, the fastener 430 may not be disposed.

The foregoing separately describes the components in the detection assembly 4, and the following continues to describe an overall assembly structure of the detection assembly 4.

FIG. 13 is a diagram of a sectional view structure of the detection assembly 4. A section C-C in FIG. 7 is used as a section in FIG. 13.

As shown in FIG. 13, the electronic component 4a may be mounted on one side of the support 44. With reference to FIG. 13 and FIG. 8, the first circuit board 41 may be connected to one side (for example, a lower side in FIG. 13) of the support 44, for example, bonded by using an adhesive. The coherent light source 46 may be located in the through hole 44b of the support 44, and the image sensor 47 may be located in the through hole 44c of the through hole support 44. In a direction from the outer side to the inner side of the cover 45, the coherent light source 46/image sensor 47, a part that is of the first circuit board 41 and that is connected to the coherent light source 46 and the image sensor 47, the force conductive member 431, the pressure sensor 432, and the second circuit board 42 are sequentially disposed.

As shown in FIG. 13, the cover 45 may be mounted on the other side (for example, an upper side in FIG. 13) of the support 44, and covers the support 44, the coherent light source 46, and the image sensor 47, so that the entire electronic component 4a is located on the inner side (that is, a side of the inner surface 45y) of the cover 45.

With reference to FIG. 8 and FIG. 13, the first light-transmitting region 45a may be aligned with the coherent light source 46, and light emitted by the coherent light source 46 may be emitted through the first light-transmitting region 45a. The second light-transmitting region 45c may be aligned with the image sensor 47, and the image sensor 47 may perform imaging on light that passes through the second light-transmitting region 45c. Herein, "aligned" means that the two overlap, including complete overlap or only partial overlap. For example, that the first light-transmitting region 45a is aligned with the coherent light source 46 may be that the first light-transmitting region 45a completely overlaps or partially overlaps the coherent light source 46. When the first light-transmitting region 45a completely overlaps the coherent light source 46, an area of the first light-transmitting region 45a may be consistent with an area of the coherent light source 46. When the first light-transmitting region 45a partially overlaps the coherent light source 46, an area of the first light-transmitting region 45a may be greater than or equal to an area of the coherent light source 46, or may be less than or equal to an area of the coherent light source 46. The foregoing explanation of alignment is also applicable below.

In an embodiment, as shown in FIG. 13, the detection assembly 4 may further include a Fresnel film 53, and the Fresnel film 53 may be disposed between the cover 45 and the coherent light source 46. The Fresnel film 53 can transmit coherent light emitted by the coherent light source 46, and can make the coherent light more concentrated or reduce scattering of the coherent light, to enhance brightness of the coherent light. The Fresnel film 53 may further block another structure near the coherent light source 46, to prevent a user from seeing the another structure from the outer side of the cover 45, so as to enhance appearance experience of the product. It may be understood that the Fresnel film 53 is not necessary.

In an embodiment, as shown in FIG. 13, the detection assembly 4 may further include a lens component 50. The lens component 50 may be disposed in the second light-transmitting region 45c, and the lens component 50 may be located between the outer surface 45x and the image sensor 47. For example, the lens component 50 may be disposed on the inner side of the cover 45. For example, the lens component 50 may be connected to the inner surface 45y. Alternatively, the lens component 50 may be embedded in the second light-transmitting region 45c, and the second light-transmitting region 45c may be an entity structure or a through hole. The lens component 50 is configured to transmit and process scattered light, so that the image sensor 47 performs imaging. The scattered light is further described below.

For example, the lens component 50 may be a micro lens or a micro lens array (microlens array). The micro lens is a single lens, and the micro lens array is formed by a plurality of very small-sized lenses that are arranged in array. The micro lens array has a thin thickness, and can better adapt to limited structural space.

In another embodiment, based on a product requirement, the lens component 50 may not be disposed.

In an embodiment, as shown in FIG. 13, the detection assembly 4 may further include a light-blocking structure. The light-blocking structure is disposed between the coherent light source 46 and the image sensor 47. The light-blocking structure is configured to block light emitted by the coherent light source 46 from directly entering the image sensor 47 along a path below the outer surface 45x of the cover 45. In other words, the light-blocking structure is configured to block light that is emitted by the coherent light source 46 and that does not pass through the outer surface 45x of the cover 45 from entering the image sensor 47.

For example, with reference to FIG. 8 and FIG. 13, the light-blocking structure may include a rib structure 44d disposed on the support 44. The rib structure 44d separates the through hole 44b from the through hole 44c, and also separates the coherent light source 46 from the image sensor 47.

For example, as shown in FIG. 13, the light-blocking structure may further include an opaque region 45b disposed on the cover 45. The opaque region 45b is a local three-dimensional region of the cover 45. The opaque region 45b may be integrally formed in the cover 45. The opaque region 45b is connected to another region of the cover 45, and may be formed in a manufacturing process of the cover 45. Alternatively, the opaque region 45b may be manufactured in advance (the opaque region 45b is an opaque part), and is assembled to the body of the cover through an assembly process, to manufacture the cover 45. The opaque region 45b may be aligned with the rib structure 44d.

In another embodiment, based on a product requirement, the rib structure 44d and/or the opaque region 45b may not be disposed.

As shown in FIG. 13, for example, the rib structure 44d may be connected between the opaque region 45b and the first circuit board 41. The cover 45, the rib structure 44d, a part that is of the first circuit board 41 and that is connected to the coherent light source 46/image sensor 47, the force conductive member 431, and the pressure sensor 432 are sequentially arranged. Therefore, the contact pressure received by the cover 45 may be fully transferred to the pressure sensor 432 through the rib structure 44d and the force conductive member 431. Therefore, the rib structure 44d may be used as a part of the force conductive structure. It may be understood that, the part that is of the first circuit board 41 and that is connected to the coherent light source 46/image sensor 47 may also conduct the contact pressure. Therefore, it may be considered that the part is also a part of the force conductive structure.

It may be understood that the rib structure 44d has both a light-blocking function and a contact pressure conduction function. This is merely an example, and is not intended to limit this embodiment of this application. In practice, the contact pressure may be conducted at any location of the detection assembly 4 by using any appropriate structure. For example, the entire support 44 may be configured to conduct the contact pressure.

In an embodiment, the wearable device 1 may have an electrocardiogram detection function. For example, the detection assembly 4 may further include a detection electrode, configured to contact a human body to collect a bioelectric signal. For example, as shown in FIG. 8, the detection electrode may be an electrically conductive layer 45d formed on a surface of the cover 45. The electrically conductive layer 45d may be formed on the outer surface 45x of the cover 45, the inner surface 45y of the cover 45, and a side surface 45z connecting the outer surface 45x and the inner surface 45y. In other words, the electrically conductive layer 45d may extend from the outer surface 45x of the cover 45 to the inner surface 45y. The electrically conductive layer 45d may avoid the first light-transmitting region 45a, the second light-transmitting region 45c, the third light-transmitting region 45e, and the like. A specific distribution and an area of the electrically conductive layer 45d may be designed based on a requirement, and are not limited to those shown in FIG. 8. For ease of touch by the user, the area of the electrically conductive layer 45d may be as large as possible.

As shown in FIG. 13, the detection assembly 4 may further include an electrically conductive member 49, the electrically conductive member 49 may be disposed on the inner side of the cover 45, and the electrically conductive member 49 may be electrically connected to the first circuit board 41. With reference to FIG. 13 and FIG. 8, the electrically conductive member 49 may be connected to a region that is of the electrically conductive layer 45d and that is located on the inner surface of the cover 45. The electrically conductive member 49 has an electrically conductive property, and may be, for example, electrically conductive silica gel. Therefore, the electrically conductive layer 45d may be electrically connected to the first circuit board 41 through the electrically conductive member 49, to establish a loop of an electrocardiosignal. It may be understood that the electrocardiosignal may include an electrocardiogram (electrocardiogram, ECG) signal.

In an embodiment, the detection assembly 4 may further detect ambient light, where the ambient light includes at least one of visible light and invisible light. The detection assembly 4 may detect, for example, at least one of parameters such as intensity, illumination, spectral distribution, and color temperature of the ambient light. Based on this, the wearable device 1 may provide information like sunshine duration, a sunshine suggestion, a vision protection suggestion, and a sun protection suggestion for the user. The following provides descriptions.

As shown in FIG. 8, for example, the cover 45 may further have a third light-transmitting region 45e, and the third light-transmitting region 45e is a three-dimensional region. There is a specific distance between the third light-transmitting region 45e and the first light-transmitting region 45a and the second light-transmitting region 45c. For example, the first light-transmitting region 45a, the second light-transmitting region 45c, and the third light-transmitting region 45e may be sequentially arranged. A distance between the first light-transmitting region 45a and the second light-transmitting region 45c may be small, and a distance between the third light-transmitting region 45e and the second light-transmitting region 45c may be large. A shape of the third light-transmitting region 45e is not limited, and FIG. 8 is merely an example. The third light-transmitting region 45e may be an entity structure. The third light-transmitting region 45e may be integrally formed in the cover 45, or may be manufactured in advance (the third light-transmitting region 45e is a part), and assembled to the body of the cover through an assembly process, to manufacture the cover 45. Alternatively, the third light-transmitting region 45e may be a through hole. The third light-transmitting region 45e may transmit light.

As shown in FIG. 8, for example, a through hole 44e may be further disposed on the support 44.

With reference to FIG. 8 and FIG. 13, the detection assembly 4 may further include an ambient light sensor 52. The ambient light sensor 52 may be disposed on the inner side of the cover 45, and is located in the through hole 44e of the support 44. The ambient light sensor 52 may correspond to the third light-transmitting region 45e of the cover 45. For example, the ambient light sensor 52 may be aligned with the third light-transmitting region 45e. Ambient light may pass through the third light-transmitting region 45e and be sensed by the ambient light sensor 52. After a signal of the ambient light sensor 52 is processed by the controller, a parameter of the ambient light may be obtained. For example, the ambient light sensor 52 may have a function of an ultraviolet sensor, and the ambient light sensor 52 may detect an ultraviolet radiation amount in the ambient light, so that the wearable device 1 may provide a sun protection suggestion for the user.

FIG. 13 does not show a circuit connection of the ambient light sensor 52. In practice, the ambient light sensor 52 may be electrically connected to the controller by using any appropriate structure. For example, a third circuit board may be specially disposed. The ambient light sensor 52 is electrically connected to the third circuit board, and the ambient light sensor 52 is electrically connected to the controller through the third circuit board. Alternatively, the ambient light sensor 52 may also be electrically connected to the second circuit board 42 or the first circuit board 41, and is electrically connected to the controller through the second circuit board 42 or the first circuit board 41.

As shown in FIG. 13, in an embodiment, the ambient light sensor 52 may be a photodiode. A light filter 51 may be further disposed in the third light-transmitting region 45e of the cover 45. For example, the light filter 51 may be disposed on an outer surface of the third light-transmitting region 45e, and the outer surface of the third light-transmitting region 45e may be deployed downward by a specific distance relative to the outer surface 45x of the cover 45. Alternatively, the light filter 51 may be disposed on an inner surface of the third light-transmitting region 45e, and the inner surface of the third light-transmitting region 45e may be deployed inward by a specific distance relative to the inner surface 45y of the cover 45. Alternatively, the light filter 51 may be embedded in the third light light-transmitting region 45e. The light filter 51 may allow light of some bands to pass through and filter light of other bands. For example, the light filter 51 may allow light of an ultraviolet band (for example, a wavelength is about 100 nm to 400 nm) to pass through, and filter light in another band, for example, visible light and infrared light. The ambient light sensor 52 corresponding to the light filter 51 may have a function of detecting an ultraviolet radiation amount. In another embodiment, the ambient light sensor 52 may be manufactured by using a material that is sensitive to light of a specific band, and there is no need to additionally dispose a light filter 51 on the cover 45, where the band may be an ultraviolet band (it may be understood that the ambient light sensor 52 may be referred to as an ultraviolet sensor). Alternatively, based on a product requirement, the ambient light sensor 52 may not be disposed in the wearable device 1, and the wearable device 1 may not have a function of detecting ambient light.

As shown in FIG. 13, for example, the coherent light source 46, the image sensor 47, and the ambient light sensor 52 may be sequentially arranged. In other words, the ambient light sensor 52 is located on a side that is of the image sensor 47 and that is away from the coherent light source 46. Based on such a layout, the coherent light source 46 may be far away from the ambient light sensor 52, so that a case in which detection precision of the ambient light is affected when the light of the coherent light source 46 directly enters the ambient light sensor 52 and is detected by the ambient light sensor 52 due to excessively closeness between the coherent light source 46 and the ambient light sensor 52 can be avoided. For example, the electrically conductive member 49 may be located between the image sensor 47 and the ambient light sensor 52, and the electrically conductive member 49 may further block the light of the coherent light source 46 from directly entering the ambient light sensor 52. It may be understood that, this is merely an example, and a location relationship between the electrically conductive member 49 and the ambient light sensor 52 is not limited in this embodiment of this application.

In an embodiment, as shown in FIG. 8 and FIG. 13, a potting adhesive 48 may be filled between the cover 45 and the support 44. The potting adhesive 48 may fill space around the coherent light source 46, the image sensor 47, the electrically conductive member 49, the ambient light sensor 52, and the like, to implement packaging protection. The wearable device 1 filled with the potting adhesive 48 may have a good waterproof feature. For example, the wearable device 1 may be a diving watch. The potting adhesive 48 may further increase structural strength and improve structural reliability.

In another embodiment, the potting adhesive 48 may not be filled.

The foregoing describes an internal assembly structure of the detection assembly 4, and the following describes an assembly structure of the detection assembly 4 and the housing 3.

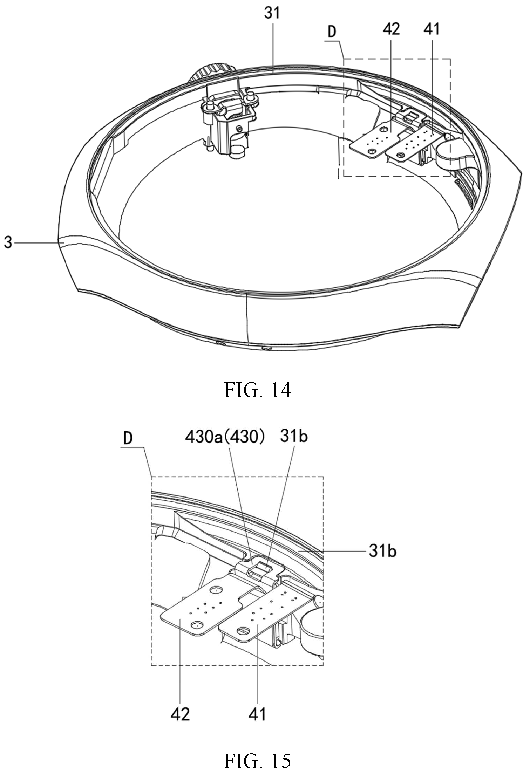

FIG. 14 and FIG. 15 are diagrams of an assembly structure of the detection assembly 4 and the side wall 31 of the housing 3. FIG. 15 is a partially enlarged diagram of a location D in FIG. 14.

With reference to FIG. 7, FIG. 9, FIG. 4, FIG. 6, and FIG. 15, the detection assembly 4 may be disposed in the through hole 31a of the side wall 31 (as described above, the through hole 31a may be a stepped hole, including the large hole 31x and the small hole 31y that are connected). The cover 45, the support 44, the coherent light source 46, the image sensor 47, the force conductive plate 431a, and the like may all be located in the large hole 31x of the through hole 31a. The connection structure 44a of the support 44 may be snap-fit with the side wall 31. The cover 45 is exposed from the side wall 31. The force conductive rod 431b penetrates the small hole 31y. The sealing ring 433 on the force conductive rod 431b may be in close contact with a hole wall of the small hole 31y to form a sealing connection. The limiting ring 434 on the force conductive rod 431b may abut against an inner surface of the side wall 31. The limiting ring 434 and the washer 435 may jointly limit the force conductive rod 431b on the side wall 31, to prevent the force conductive rod 431b from being detached from the side wall 31. The pressure sensor 432 and the second circuit board 42 may be located on the side wall 31. The first wall 430a and the third wall 430c of the fastener 430 may be separately snap-fit with the buckle 31b on the inner side of the side wall 31 (due to limitation of an angle of view, FIG. 15 shows only a snap-fit connection between the first wall 430a and the buckle 31b), so that the pressure sensor 432 and the force conductive member 431 are fastened to the side wall 31, to avoid shaking of a force conductive structure with a large volume and weight, and ensure assembly reliability and structural strength of the force conductive structure.

In this embodiment of this application, because the coherent light source 46 and the image sensor 47 are disposed, the detection assembly 4 may monitor blood flow information. Blood flow generally refers to a flow status of blood in blood vessels in a body, is a basic parameter of a blood circulation system, and reflects a process of blood flowing through arteries, capillaries and veins under a blood pumping action of a heart. The blood flow may be represented by using a plurality of parameters, including but not limited to a speed, a flow rate, a direction, stability (whether the blood flow in the circulatory system is stable), resistance (the blood flow is subject to resistance of a blood vessel wall), pulsation (a fluctuation of the blood flow in a heart pulsation process), distribution (a distribution of the blood flow in the body), and the like. This blood flow information is measured and analyzed to evaluate functions of a heart and blood vascular system, diagnose cardiovascular disease, and develop effective treatment.

The detection assembly 4 in this embodiment of this application may be further configured to monitor other physiological feature information, including but not limited to one or more of a heart rate, blood oxygen, blood pressure, breathing, emotion, an electrocardiosignal, and the like.

The blood flow information and other physiological features such as a heart rate, blood oxygen, and breathing are associated and affect each other. During physiological feature monitoring, when the information is comprehensive considered, it helps to more accurately understand a physiological state and health of the body.

Because the ambient light sensor 52 is disposed, the detection assembly 4 may further detect the ambient light. Because the pressure sensor 432 is disposed, the detection assembly 4 may further detect the contact pressure.

The following describes a detection principle of the detection assembly 4.

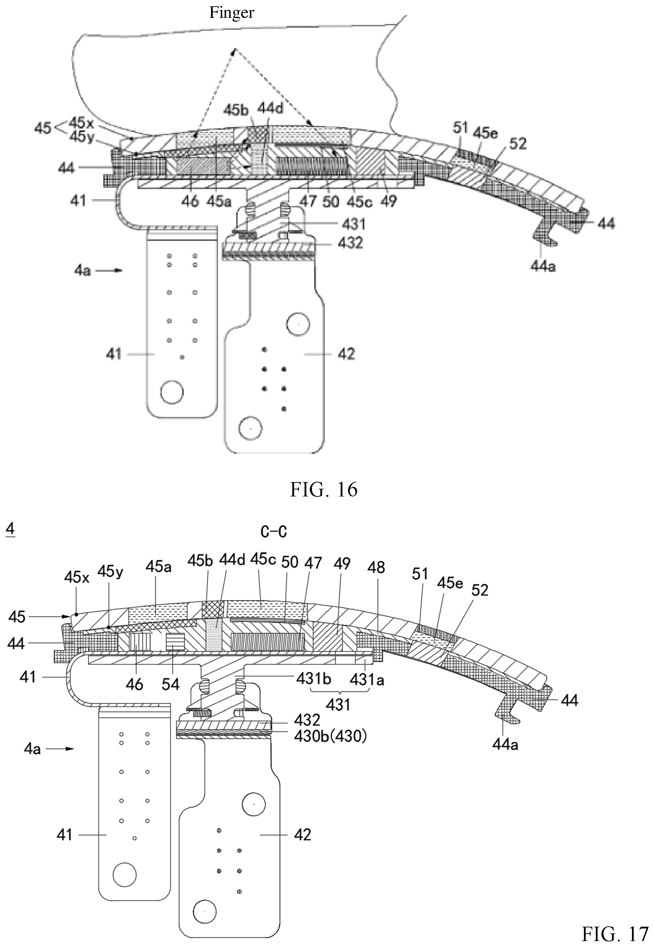

As shown in FIG. 16, the cover 45 of the detection assembly 4 is configured to be touched by a human body, to monitor a physiological feature. The following uses an example in which a finger touches the cover 45 for description.

As shown in FIG. 16, when a physiological feature needs to be monitored, the user may place the finger on the outer surface 45x of the cover 45, so that the finger covers the first light-transmitting region 45a and the second light-transmitting region 45c. The controller may control the coherent light source 46 to emit coherent light. When there are a plurality of coherent light sources 46, the controller may control the coherent light sources 46 to sequentially emit light. For example, the controller may control the coherent light source 46 to be lit once in one sampling period (for example, 10 ms), or the controller may control the coherent light source 46 to be repeatedly lit in a plurality of sampling periods. When there are a plurality of coherent light sources 46, the controller may control the coherent light sources 46 to be sequentially lit in one sampling period.

As shown in FIG. 16, coherent light emitted by the coherent light source 46 may be emitted into the finger through the first light-transmitting region 45a of the cover 45, and the coherent light emitted into the finger forms a speckle. The coherent light is scattered in finger tissue, and is emitted into the second light-transmitting region 45c through skin. Light that is scattered by the tissue and penetrates the skin may be referred to as scattered light. When there are a plurality of coherent light sources 46 with different wavelengths, coherent light of different wavelengths is emitted into the finger tissue at different depths.

When the lens component 50 is disposed in the second light-transmitting region 45c, the scattered light passes through the lens component 50, and the lens component 50 images the speckle onto the image sensor 47.

The image sensor 47 can sense an optical signal to generate an electrical signal, to implement imaging. When there are a plurality of coherent light sources 46, for each coherent light source 46, the image sensor 47 may generate a corresponding image. For example, in each sampling period, the image sensor 47 may generate a corresponding image for each coherent light source 46. The image sensor 47 may detect a laser Doppler (laser doppler) effect caused by blood flow, so that a blood flow change in a pulse fluctuation process can be detected.

The controller processes the image generated by the image sensor 47, and may generate blood flow information and other physiological feature information. For example, for coherent light emitted by a coherent light source 46 in a green light band, the controller may generate a heart rate parameter after processing. For coherent light emitted by a coherent light source 46 in a red light band and a near-infrared light band, the controller may generate a blood oxygen parameter after processing. Therefore, the plurality of coherent light sources 46 are disposed, so that blood flow information and more physiological feature information can be monitored. It may be understood that, when one coherent light source 46 is disposed, blood flow information and less physiological feature information may be monitored.

For example, for an image formed by each coherent light source 46 in one sampling period, the controller may perform sliding window processing, to generate a speckle plethysmography (speckle plethysmography, SPG) signal, so as to monitor blood flow information and other physiological feature information. The sliding window processing refers to sliding a sliding window of a fixed size on a data set, to perform statistical processing on data points in the sliding window. The sliding window processing may include the following steps: selecting the sliding window of the fixed size (for example, 10*10 pixels) on the data set; performing statistical processing on the data points in the sliding window, where the statistical processing includes but is not limited to mean processing, standard deviation processing, and the like; moving the sliding window along image space, and performing new statistical processing after each movement, to generate a series of data points including blood flow information and other physiological feature information.

A parameter K representing the blood flow information (for example, a flow rate) may be obtained by performing statistical processing on each sliding window. For example, the parameter K = standard deviation/average value. A plurality of parameters K may be obtained by performing statistical processing on all sliding windows in images formed by coherent light sources 46 in one sampling period, and the plurality of parameters K may form a K value curve corresponding to each coherent light source 46. In a plurality of sampling periods, imaging and processing are repeatedly performed, so that a plurality of K value curves of each coherent light source 46 at different time points may be obtained. The plurality of K value curves of each coherent light source 46 at different time points may reflect a blood flow change under a pulse fluctuation, to form an SPG signal. As described above, the SPG signal may represent the blood flow information.

In this embodiment, an amplitude of the SPG signal may be used as a conventional photo plethysmography (photo plethysmography, PPG) signal, and the PPG signal may represent a physiological feature like a heart rate, blood oxygen, blood pressure, breathing, and emotion. In other words, after sliding window processing, both the SPG signal and the PPG signal may be generated, and a combination of the SPG signal and the PPG signal may effectively monitor blood flow information and a physiological feature like a heart rate, blood oxygen, blood pressure, breathing, and emotion.

In another embodiment, the controller may further process the image generated by the image sensor 47 in any appropriate manner. This is not limited to the sliding window processing described above.

As shown in FIG. 16, when the light-blocking structure like the opaque region 45b and the rib structure 44d is disposed between the coherent light source 46 and the image sensor 47, the light-blocking structure may prevent the light emitted by the coherent light source 46 from reaching the image sensor 47 from the inner side of the cover 45 or through the inside of the cover 45. It can be ensured that the coherent light can be collected by the image sensor 47 after being scattered by finger tissue but the coherent light does not directly enter the image sensor 47. For example, FIG. 16 shows several optical paths of coherent light that is emitted by the coherent light source 46 and that does not pass through the outer surface 45x of the cover 45. The light-blocking structure may block the coherent light on the optical paths. The design can ensure detection precision of the physiological feature information.

As shown in FIG. 16, when a finger touches the cover 45, contact pressure exerted by the finger to the cover 45 may be transferred to the pressure sensor 432 through the electrically conductive member 49, the force conductive member 431, and the like, and the pressure sensor 432 senses the contact pressure to generate an electrical signal. The controller performs comprehensive processing on the signal of the pressure sensor 432 and the image generated by the image sensor 47, to eliminate interference caused by unstable pressing to imaging of the image sensor 47, reduce or avoid impact of motion artifacts caused by unstable pressing on physiological feature monitoring, and improve detection precision of a physiological feature.

With reference to FIG. 8 and FIG. 16, when a finger touches the electrically conductive layer 45d on the surface of the cover 45, the electrically conductive layer 45d may collect an electrical signal of a human body. The electrical signal is transmitted to the first circuit board 41 through the electrically conductive member 49, and is finally processed by the controller to obtain an electrocardiosignal, so that the wearable device 1 may have an electrocardiogram detection function.

For coherent light of each wavelength, a two-dimensional image generated by the image sensor 47 may represent a material distribution feature of finger skin from a shallow layer to a deep layer (for example, about 1 mm). For example, a penetration depth of near-infrared light is large, and a distribution image of capillaries or small blood vessels may be obtained; and a penetration depth of blue light is small, and a surface tissue image may be obtained. A material distribution feature at each depth may be used as an identity feature of the user, and the user identity may be more accurately represented based on material distribution features at a plurality of depths.

Therefore, in an embodiment, a coherent light source 46 with a plurality of wavelengths is disposed, so that the image sensor 47 generates different two-dimensional images for light of different wavelengths, and the two-dimensional images may be used to perform identity recognition for a user. For example, the two-dimensional image of the user may be collected via the image sensor 47, and the two-dimensional image is pre-stored in the wearable device 1 as a template. When identity recognition needs to be performed, the controller may compare a to-be-verified two-dimensional image currently collected by the image sensor 47 with the template. If the to-be-verified two-dimensional image matches the template, identity recognition succeeds. The template and the to-be-verified two-dimensional image may be collectively referred to as identity recognition information. The controller may process the image generated by the image sensor 47, to generate the identity recognition information.

For example, based on different recognition scenarios, when a to-be-verified two-dimensional image is collected, a part of coherent light sources 46 may emit light (for example, one coherent light source 46 emits light), so that the image sensor 47 performs imaging on only light that is emitted by the part of coherent light sources 46 and that is scattered back by human body tissue; or all coherent light sources 46 emit light, so that the image sensor 47 performs imaging on all light that is emitted by all the coherent light sources 46 and that is scattered back by human body tissue. The former case has low identification precision, and may be applied to a scenario having a low security level requirement, for example, unlocking the wearable device 1. The latter case has high identification precision, and may be applied to a scenario having a high security level requirement, for example, electronic payment.

It may be understood that a solution of performing identity recognition based on the two-dimensional image generated by the image sensor 47 is not necessary.

As shown in FIG. 16, the ambient light may pass through the third light-transmitting region 45e of the cover 45 and be collected by the ambient light sensor 52. The controller may process a signal of the ambient light sensor 52 to obtain a parameter of the ambient light. For example, the controller may specifically perform sliding window processing at a low sampling rate (for example, 1 Hz) on the signal of the ambient light sensor 52. In this embodiment, because the ambient light sensor 52 is disposed on the side wall 31 of the wearable device 1, when the user normally wears the wearable device 1, the ambient light sensor 52 can detect the ambient light, and the wearable device 1 does not need to be removed from a human body. Therefore, an operation can be simplified, and user experience can be improved. In addition, the ambient light sensor 52, the coherent light source 46, and the image sensor 47 are all disposed on the side wall 31, so that the wearable device 1 can detect both the ambient light and the physiological feature.

With reference to FIG. 8 and FIG. 16, the distance between the first light-transmitting region 45a and the second light-transmitting region 45c is small, so that a relative location of the coherent light source 46 and the image sensor 47 can match an optical path of the scattered light used to detect the physiological feature, and physiological feature monitoring can be reliably and accurately implemented. The distance between the third light-transmitting region 45e and the second light-transmitting region 45c may be large, so that a risk that light emitted by the coherent light source 46 enters the ambient light sensor 52 can be reduced, interference caused by the coherent light source 46 to the ambient light sensor 52 can be reduced, and detection precision of the ambient light can be improved.

Compared with a conventional solution, the wearable device 1 in this embodiment of this application can monitor blood flow information and other physiological feature information, can detect a large quantity of physiological features in a non-intrusive measurement manner, and can further detect ambient light. Therefore, more functions can be integrated into the wearable device 1, and miniaturization of the product can be ensured.

A button, for example, a crown, that is operated by the user may be disposed on the side wall 31 of the wearable device 1. The detection assembly 4 is disposed on the side wall 31 of the wearable device 1, so that the detection assembly 4 and the button can be arranged together, to facilitate a user operation and enhance user experience. In addition, when the detection assembly 4 is disposed on the side wall 31, the user may use a finger to touch the cover 45 to implement physiological feature monitoring, improving detection accuracy of the physiological feature monitoring of the finger.

Based on the embodiment shown in FIG. 16, in another embodiment, a light-emitting diode (LED) may be further disposed to monitor other physiological feature information, to implement a low-cost and low-power solution. The following provides descriptions.

FIG. 17 shows an assembly cross-sectional structure of a detection assembly 4 according to another embodiment. In the embodiment shown in FIG. 17, the detection assembly 4 may further include at least one light-emitting diode 54. When there are a plurality of light-emitting diodes 54, wavelengths of the light-emitting diodes 54 may be different from each other, for example, may be a red light band, a blue light band, a yellow light band, and the like. The light-emitting diode 54 and the coherent light source 46 may be arranged side by side, and both the light-emitting diode 54 and the coherent light source 46 may be disposed on the inner side of the cover 45. The light-emitting diode 54 and the coherent light source 46 may be collectively referred to as a light source. The light-emitting diode 54 may be aligned with the first light-transmitting region 45a of the cover 45, and incoherent light emitted by the light-emitting diode 54 may be emitted through the first light-transmitting region 45a. The light-emitting diode 54 may be disposed on the first circuit board 41.

In the embodiment shown in FIG. 17, a wavelength of the coherent light source 46 may be, for example, a near-infrared band and/or a red light band. A penetration depth of coherent light emitted by the coherent light source 46 in the tissue is large. The coherent light source 46 may provide an SPG signal, and the SPG signal may be used to monitor blood flow information. The incoherent light emitted by the light-emitting diode 54 may enter finger tissue after passing through the first light-transmitting region 45a. After being scattered by the tissue, the incoherent light may pass through the second light-transmitting region 45c and be collected by the image sensor 47. After the image generated by collecting the incoherent light by the image sensor 47 is processed by the controller, a PPG signal may be obtained. The PPG signal is used to represent another physiological feature like a heart rate, blood oxygen, blood pressure, breathing, and emotion.

In this embodiment, after the light-emitting diode 54 is disposed, a small quantity of coherent light sources 46 may be disposed. For example, only one coherent light source 46 is disposed. In comparison with a solution in which blood flow information and another physiological feature are monitored by using a plurality of coherent light sources 46, in this embodiment, a coherent light source 46 with high costs and power consumption is replaced by a light-emitting diode 54 with low costs and power consumption, so that costs and power consumption can be reduced. In addition, a light source combining the coherent light source 46 and the light-emitting diode 54 may also increase flexibility of a structure layout, to meet a product requirement.

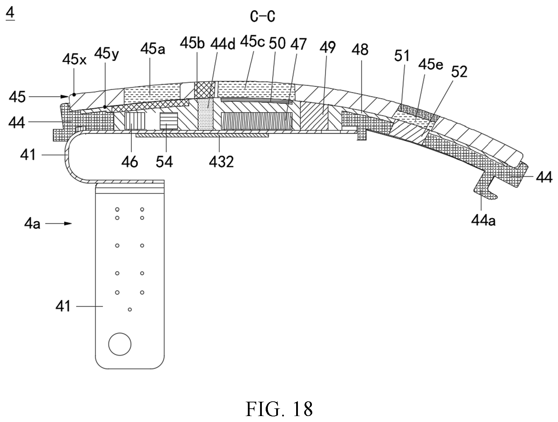

Based on the foregoing embodiment, as shown in FIG. 18, in an embodiment, the fastener 430, the force conductive member 431, and the sealing ring 433, the limiting ring 434, and the washer 435 that are mounted on the force conductive member 431 may not be disposed in the detection assembly 4. The pressure sensor 432, the image sensor 47, and the light source may all be electrically connected to the first circuit board 41. For example, the pressure sensor 432, the image sensor 47, and the light source may all be disposed on the first circuit board 41. The rib structure 44d and the like may be used as a force conductive structure to conduct contact pressure exerted on the cover 45 to the pressure sensor 432. In this embodiment, the force conductive structure is simplified, and a volume and a weight of the detection assembly can be reduced, to meet a product requirement.

As shown in FIG. 18, in an embodiment, the image sensor 47 and the light source may be disposed on one side (for example, an upper side in FIG. 18) of a thickness direction of the first circuit board 41, and the pressure sensor 432 may be disposed on the other side (for example, a lower side in FIG. 18) of the thickness direction of the first circuit board 41. In this way, the image sensor 47/the light source and the pressure sensor 432 may be separately disposed, so that a device layout on the first circuit board 41 can be simplified. The first circuit board 41 may be a deformable flexible circuit board. Therefore, the rib structure 44d and the like may transfer contact pressure to the pressure sensor 432 through the first circuit board 41.

In another embodiment, the pressure sensor 432, the image sensor 47, and the light source may be located on a same side of the first circuit board 41. The pressure sensor 432, the image sensor 47, and the light source may be arranged side by side, or the pressure sensor 432 and the image sensor 47 or the light source may be arranged in a stacked manner.

It may be understood that the detection assembly 4 shown in FIG. 18 is merely an example, and is not intended to limit this embodiment. For example, the light-emitting diode 54 may not be disposed on the detection assembly 4 in FIG. 18; and the light filter 51 may not be disposed, and an ambient light sensor 52 that is sensitive to light of some bands is used.

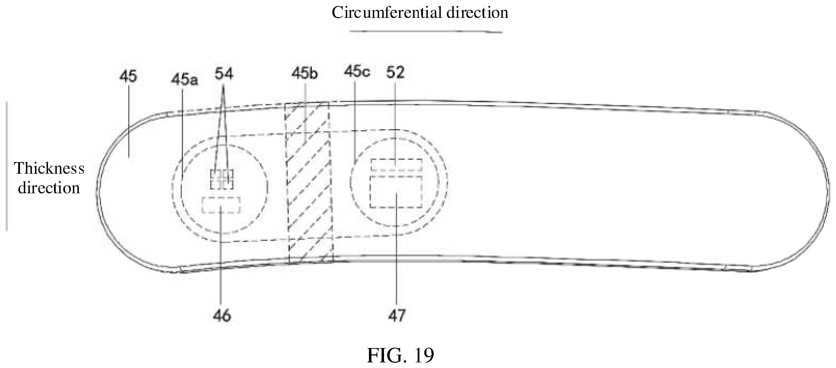

Based on the foregoing embodiment, FIG. 19 is a diagram of a layout of a coherent light source 46, a light-emitting diode 54, an image sensor 47, and an ambient light sensor 52 according to another embodiment.

As shown in FIG. 19, the coherent light source 46 and the light-emitting diode 54 may be arranged nearby, and the coherent light source 46 and the light-emitting diode 54 may be stacked approximately in a thickness direction of the wearable device 1. The ambient light sensor 52 and the image sensor 47 may be arranged nearby, and the ambient light sensor 52 and the image sensor 47 may be stacked approximately in the thickness direction. In this embodiment, the third light-transmitting region 45e is not disposed on the cover 45. Both the ambient light sensor 52 and the image sensor 47 may be aligned with the second light-transmitting region 45c of the cover 45. The ambient light sensor 52 may detect ambient light that passes through the second light-transmitting region 45c. In this embodiment, the structure layout of the coherent light source 46, the light-emitting diode 54, the image sensor 47, and the ambient light sensor 52 can be optimized, so that a size of the detection assembly 4 in a circumferential direction is reduced. In this way, the detection assembly 4 is more compact, so that a size of the wearable device 1 is further reduced. The circumferential direction may be a direction surrounding the thickness square axis of the wearable device 1 in FIG. 2.

It may be understood that, in FIG. 19, a relative location relationship between the coherent light source 46, the light-emitting diode 54, the image sensor 47, and the ambient light sensor 52 is merely an example, and is not intended to limit this embodiment. In practice, locations of the foregoing devices may be flexibly adjusted based on a product requirement. For example, with reference to FIG. 19, the coherent light source 46 and the light-emitting diode 54 may exchange locations, and the image sensor 47 and the ambient light sensor 52 may exchange locations. The light source may be disposed on a right side of the opaque region 45b, and the image sensor 47 and the ambient light sensor 52 may be disposed on a left side of the opaque region 45b.

In the foregoing embodiments, an example in which the detection assembly 4 is disposed on the side wall 31 of the wearable device 1 is used. In another embodiment, the detection assembly 4 may alternatively be disposed on any side of the wearable device 1, for example, may be disposed on a bottom wall of the wearable device 1. The bottom wall is opposite to the display 2.

An embodiment of this application further provides a detection method, where the detection method may be applied to the foregoing wearable device 1. The detection method may include:
controlling a light source in the detection assembly 4 to emit light, where the light source includes the coherent light source 46, or includes the coherent light source 46 and the light-emitting diode 54;
controlling the image sensor 47 to perform imaging on light that passes through the second light-transmitting region 45c; and
processing the image generated by the image sensor 47, to generate blood flow information and further generate other physiological feature information.

The foregoing detection process may be triggered under a specific condition. For example, when the user opens a built-in application of the wearable device 1 and selects a detection option, detection may be triggered. Alternatively, when the user touches the detection electrode on the cover 45, the detection is triggered when the detection electrode collects a bioelectric signal of the human body. Alternatively, the detection assembly 4 may remain in a standby state in a specified time period. When the user touches the first light-transmitting region 45a on the cover 45, light emitted by the light source is scattered by human body tissue to the image sensor 47, so that the detection assembly 4 performs detection.

An embodiment of this application further provides an electronic device, including: one or more controllers, and a non-transitory computer-readable storage medium that is coupled to the controller and stores a program executed by the controller. When the program is executed by the controller, the electronic device performs the detection method. The electronic device includes but is not limited to the wearable device 1, and may further be, for example, a device like a mobile phone, a tablet computer, a notebook computer, a personal digital assistant (personal digital assistant, PDA), or a vehicle-mounted device.

An embodiment of this application further provides a non-transitory computer-readable storage medium, including program code. When the program code is executed by a computer device, the computer device is enabled to perform the detection method.

An embodiment of this application further provides a chip, including: a controller. The controller is configured to invoke a computer program from a memory and run the computer program, so that a device in which the chip is mounted performs the detection method.

It should be understood that the controller in embodiments of this application may be a central processing unit (Central Processing Unit, CPU), or may be another general-purpose processor, a digital signal processor (Digital Signal Processor, DSP), an application-specific integrated circuit (Application-Specific Integrated Circuit, ASIC), a field programmable gate array (Field Programmable Gate Array, FPGA) or another programmable logic device, a discrete gate or transistor logic device, a discrete hardware component, or the like. The general-purpose processor may be a microprocessor or any regular processor or the like.

It may be understood that the memory in embodiments of this application may be a volatile memory or a non-volatile memory, or may include a volatile memory and a non-volatile memory. The non-volatile memory may be a read-only memory (Read-Only Memory, ROM), a programmable read-only memory (Programmable ROM, PROM), an erasable programmable read-only memory (Erasable PROM, EPROM), an electrically erasable programmable read-only memory (Electrically EPROM, EEPROM), or a flash memory. The volatile memory may be a random access memory (Random Access Memory, RAM) that is used as an external cache. By way of example but not limitative description, RAMs in many forms may be used, for example, a static random access memory (Static RAM, SRAM), a dynamic random access memory (Dynamic RAM, DRAM), a synchronous dynamic random access memory (Synchronous DRAM, SDRAM), a double data rate synchronous dynamic random access memory (Double Data Rate SDRAM, DDR SDRAM), an enhanced synchronous dynamic random access memory (Enhanced SDRAM, ESDRAM), a synchlink dynamic random access memory (Synchlink DRAM, SLDRAM), and a direct rambus random access memory (Direct Rambus RAM, DR RAM).

It should be noted that when the controller is a general-purpose processor, a DSP, an ASIC, an FPGA, another programmable logic device, a discrete gate or a transistor logic device, or a discrete hardware component, the memory (storage module) may be integrated into the controller.

It should be noted that the memory described in this specification is intended to include but not limited to these memories and any memory of another appropriate type.

A person of ordinary skill in the art may be aware that, in combination with units and algorithm steps of the examples described in embodiments disclosed in this specification, this application can be implemented by electronic hardware or a combination of computer software and electronic hardware. Whether the functions are performed by hardware or software depends on particular applications and design constraints of the technical solution. A person skilled in the art may use different methods to implement the described functions for each particular application, but it should not be considered that the implementation goes beyond the scope of this application.

In the several embodiments provided in this application, it should be understood that the disclosed system, apparatus, and method may be implemented in other manners. For example, the described apparatus embodiments are merely examples. For example, division into the units is merely logical function division. There may be another division manner during actual implementation. For example, a plurality of units or components may be combined or integrated into another system, or some features may be ignored or not performed. In addition, the displayed or discussed mutual couplings or direct couplings or communication connections may be implemented through some interfaces. The indirect couplings or communication connections between the apparatuses or units may be implemented in an electrical form, in a mechanical form, or in another form.

The foregoing units described as separate parts may or may not be physically separate, and parts displayed as units may or may not be physical units, may be located in one position, or may be distributed on a plurality of network units. Some or all of the units may be selected based on actual requirements to achieve the objectives of the solutions of embodiments.

In addition, function units in embodiments of this application may be integrated into one processing unit, each of the units may exist alone physically, or two or more units are integrated into one unit.

When the functions are implemented in a form of software functional unit and sold or used as an independent product, the functions may be stored in a computer readable storage medium. Based on such an understanding, the technical solutions in this application essentially, or the part contributing to the conventional technology, or some of the technical solutions may be implemented in a form of software product. The computer software product is stored in a storage medium, and includes several instructions for instructing a computer device (which may be a personal computer, a server, or a network device) to perform all or some of the steps of the methods described in embodiments of this application. The foregoing storage medium includes: any medium that can store program code, such as a USB flash drive, a removable hard disk, a read-only memory (Read-Only Memory, ROM), a random access memory (Random Access Memory, RAM), a magnetic disk, or an optical disc.

In descriptions of embodiments of this application, unless otherwise specified, "a plurality of" means two or more.

Terms such as "first" and "second" are used only for description purposes, and cannot be understood as an indication or implication of relative importance or an implicit indication of a quantity of indicated technical features. A feature limited by "first" or "second" may explicitly or implicitly include one or more features.

"Connection" should be understood in a broad sense. For example, "connection" may be a detachable connection, a nondetachable connection, a direct connection, or an indirect connection through an intermediate medium. "Fastening" should also be understood in a broad sense. For example, "fastening" may be direct fastening or indirect fastening through an intermediate medium.

The orientation terms mentioned in embodiments of this application, for example, "upper", "lower", "front", "rear", "left", "right", "inside", "outside", "side", "top", and "bottom" are merely directions with reference to the accompanying drawings. The orientation terms are used to better and more clearly describe and understand embodiments of this application, rather than explicitly or implicitly indicating that an indicated apparatus or element needs to have a specific orientation or be constructed and operated in a specific orientation, and therefore cannot be construed as a limitation on embodiments of this application.

In the descriptions of embodiments of this application, unless otherwise specified, "and/or" is merely an association relationship for describing an associated object, and indicates that three relationships may exist. For example, A and/or B may represent the following three cases: Only A exists alone, both A and B exist, and only B exists.

The foregoing descriptions are merely specific implementations of this application, but are not intended to limit the protection scope of this application. Any variation or replacement readily figured out by a person skilled in the art within the technical scope disclosed in this application shall fall within the protection scope of this application. Therefore, the protection scope of this application shall be subject to the protection scope of the claims.

## Claims

1. A detection assembly, wherein
the detection assembly comprises a cover, a coherent light source, and an image sensor;
the cover is configured to be disposed on a side wall of a housing of a wearable device, the cover has an outer surface and an inner surface opposite to the outer surface, the outer surface is exposed from the side wall, and the cover has a first light-transmitting region and a second light-transmitting region;
the coherent light source is disposed on a side of the inner surface of the cover, and the coherent light source is configured to emit coherent light that passes through the first light-transmitting region; and
the image sensor is disposed on the side of the inner surface of the cover, and the image sensor is configured to perform imaging on light that passes through the second light-transmitting region.

2. The detection assembly according to claim 1, wherein
there are at least two coherent light sources, and the at least two coherent light sources are configured to separately transmit coherent light of different wavelengths.

3. The detection assembly according to claim 1 or 2, wherein
the detection assembly further comprises a light-emitting diode, wherein the light-emitting diode is disposed on the side of the inner surface of the cover, and the light-emitting diode is configured to emit incoherent light that passes through the first light-transmitting region; and
the image sensor is further configured to perform imaging on the light that passes through the second light-transmitting region.

4. The detection assembly according to any one of claims 1 to 3, wherein
the detection assembly further comprises a light-blocking structure, wherein the light-blocking structure is disposed between a light source and the image sensor, the light-blocking structure is configured to block light that is emitted by the light source and that does not pass through the outer surface of the cover from entering the image sensor, and the light source comprises the coherent light source, or comprises the coherent light source and the light-emitting diode.

5. The detection assembly according to claim 4, wherein
the light-blocking structure comprises an opaque region of the cover.

6. The detection assembly according to any one of claims 1 to 5, wherein
the detection assembly further comprises a pressure sensor, and the pressure sensor is disposed on the side of the inner surface of the cover.

7. The detection assembly according to claim 6, wherein
the detection assembly further comprises a force conductive structure, the force conductive structure is disposed between the cover and the pressure sensor, and the force conductive structure is configured to conduct, to the pressure sensor, contact pressure exerted on the cover.

8. The detection assembly according to claim 6 or 7, wherein
the detection assembly further comprises a circuit board disposed on the side of the inner surface of the cover, wherein the pressure sensor, the light source, and the image sensor are all electrically connected to the circuit board, and the light source comprises the coherent light source, or comprises the coherent light source and the light-emitting diode.

9. The detection assembly according to claim 8, wherein
the light source and the image sensor are disposed on one side of a thickness direction of the circuit board, and the pressure sensor is disposed on the other side of the thickness direction of the circuit board.

10. The detection assembly according to any one of claims 1 to 9, wherein
the detection assembly further comprises an ambient light sensor, wherein the ambient light sensor is disposed on the side of the inner surface of the cover; and
the ambient light sensor is configured to detect ambient light that passes through the second light-transmitting region; or the cover has a third light-transmitting region, and the ultraviolet sensor is configured to detect ambient light that passes through the third light-transmitting region.

11. The detection assembly according to claim 10, wherein
the detection assembly further comprises a light filter, the light filter is disposed in the second light-transmitting region or the third light-transmitting region, and the light filter corresponds to the ambient light sensor.

12. The detection assembly according to claim 10 or 11, wherein
the light source, the image sensor, and the ambient light sensor are sequentially disposed, wherein the light source comprises the coherent light source, or comprises the coherent light source and the light-emitting diode.

13. The detection assembly according to any one of claims 1 to 12, wherein
the detection assembly further comprises a lens component, and the lens component is disposed in the second light-transmitting region.

14. The detection assembly according to claim 13, wherein
the lens component is disposed on the side of the inner surface of the cover.

15. The detection assembly according to any one of claims 1 to 14, wherein
the detection assembly further comprises an electrically conductive layer and an electrically conductive member; the electrically conductive layer is disposed on the outer surface of the cover, the inner surface of the cover, and a side surface connecting the outer surface and the inner surface; and the electrically conductive member is disposed on the side of the inner surface of the cover, and the electrically conductive member is connected to a region that is of the electrically conductive layer and that is located on the inner surface.

16. A wearable device, comprising
a housing and the detection assembly according to any one of claims 1 to 15, wherein the housing comprises a side wall, the detection assembly is disposed on the side wall, and the cover of the detection assembly is exposed from the side wall.

17. The wearable device according to claim 16, wherein
the wearable device further comprises a display, the display is disposed on the housing, and the side wall surrounds the display.

18. The wearable device according to claim 16 or 17, wherein
the wearable device further comprises a controller, the controller is disposed in the housing, and the controller is configured to: control a coherent light source in the detection assembly to emit light, control the image sensor to perform imaging on light that passes through the second light-transmitting region, process an image generated by the image sensor, and generate blood flow information.

19. The wearable device according to claim 18, wherein
the controller is further configured to: control a light-emitting diode in the detection assembly to emit light, control the image sensor to perform imaging on the light that passes through the second light-transmitting region, process the image generated by the image sensor, and generate other physiological feature information.

20. The wearable device according to claim 18 or 19, wherein
the controller is further configured to: process the image generated by the image sensor, and generate identity recognition information.
